## Europäisches Patentamt

## European Patent Office

## Office européen des brevets

(11) Veröffentlichungsnummer: **0 098 893 B1**

(12) **EUROPÄISCHE PATENTSCHRIFT**

(45) Veröffentlichungstag der Patentschrift:
07.10.87

(51) Int. Cl.⁴: **A 61 M 5/14**

(21) Anmeldenummer: 82106321.1

(22) Anmeldetag: 15.07.82

(54) **Vorrichtung zum Applizieren flüssiger Arzneimittel.**

(43) Veröffentlichungstag der Anmeldung:
**25.01.84 Patentblatt 84/4**

(73) Patentinhaber: **Ferring Biotechnik GmbH, Wittland 11-13, D-2300 Kiel 1 (DE)**

(45) Bekanntmachung des Hinweises auf die Patenterteilung:
**07.10.87 Patentblatt 87/41**

(72) Erfinder: **Kölln, Harm, Fallreep 43, D-2300 Kiel-Schilksee (DE)**

(84) Benannte Vertragsstaaten:
**AT BE CH DE FR GB IT LI LU NL SE**

(74) Vertreter: **Türk, Gille, Hrabal, Bruckner Strasse 20, D-4000 Düsseldorf 13 (DE)**

(56) Entgegenhaltungen:
**WO - A - 80/01755**
**CH - A - 461 034**
**DE - A - 1 961 761**
**FR - A - 2 305 197**
**FR - A - 2 370 481**
**US - A - 2 842 123**
**US - A - 4 332 246**

ACTORUM AG

# Beschreibung

Die Erfindung betrifft eine Vorrichtung zum Applizieren flüssiger Arzneimittel, die einen das zu applizierende Arzneimittel aufnehmenden flexiblen Beutel und eine von diesem Beutel ausgehende Leitung zum Abgeben des Arzneimittels aufweist, wobei der Beutel in einem druckdichten, mit einem unter Druck stehenden Gas zu füllenden Gehäuse angeordnet und jeweils ein Steuerventil zum Steuern des Gasdruckes im Gehäuse bzw. der aus dem Beutel abgegebenen Menge des Arzneimittels in der Leitung aufweist.

Bei einer bekannten Vorrichtung zum intermittierenden pulsatorischen Applizieren flüssiger Arzneimittel (DE-A-30 15 777) ist der das Arzneimittel aufnehmende Beutel zusammen mit einer Schlauchpumpe, dem Antriebsmotor für die Schlauchpumpe, einer Steuerung und einer anzahl von elektrischen Batterien in einem kastenartigen Gehäuse untergebracht, das beispielsweise an einem Gürtel unter der Oberbekleidung getragen werden kann. Diese vorbekannte Vorrichtung arbeitet zwar funktionsmässig äusserst zufriedenstellend, hat jedoch den Nachteil, dass sie verhältnismässig schwer und auch kompliziert und dementsprechend teuer ist. Diese Nachteile sind insbesondere auf die Verwendung der Schlauchpumpe zurückzuführen, die einen Antriebsmotor und entsprechend gross ausgelegte Batterien benötigt. Auch die elektronische Steuerung dieser vorbekannten Vorrichtung ist verhältnismässig raumaufwendig.

Die US-A-2 842 123 offenbart eine Vorrichtung zum Applizieren von Flüssigkeiten wie Blut, Plasma, Salzlösungen und dergleichen, d.h. sie ist auch zum Applizieren flüssiger Arzneimittel geeignet. In einem Gehäuse kann ein steriler flexibler Behälter für Transfusionsflüssigkeit untergebracht werden, wobei zum Applizieren dieser Flüssigkeit Druckmittel wie Gas oder Luft in den Behälter eingeleitet wird, um den Behälter bzw. Beutel entsprechend der gewünschten Menge der abzugebenden Flüssigkeit unter Druck zu setzen. Sowohl die Druckmittelquelle als auch die Ventile zum Steuern der Gaszufuhr und der aus dem Beutel ausfliessenden Flüssigkeit sind ausserhalb des Gehäuses angeordnet. Daher ist diese vorbekannte Vorrichtung nur für stationäre Behandlung geeignet, d.h. sie lässt sich nicht als tragbare Vorrichtung ausbilden und noch viel weniger einem Patienten implantieren.

Der Erfindung liegt die Aufgabe zugrunde, eine Vorrichtung zum Applizieren flüssiger Arzneimittel zu schaffen, die einfach aufgebaut und leicht ausgebildet ist, so dass sie sehr viel weniger Raum als vorbekannte Vorrichtungen dieser Art benötigt und daher auch implantiert werden kann.

Diese Aufgabe wird erfindungsgemäss dadurch gelöst, dass im druckdichten Gehäuse ein Vorratsbehälter für Druckgas, der ein steuerbares Auslassventil aufweist, und die Steuerventile angeordnet sind. Durch Öffnen des Ventiles kann man den Gasdruck im druckdichten Gehäuse aufbauen und regulieren. Dieser Gasdruck wirkt unmittelbar auf den ebenfalls im druckdichten Gehäuse untergebrachten flexiblen Beutel, wodurch das in dem Beutel befindliche Arzneimittel aus der an den Beutel angeschlossenen Leitung ausgedrückt wird, wenn dem nicht ein Widerstand entgegengesetzt ist, beispielsweise indem ein in oder an der Leitung vorgesehenes Ventil geschlossen oder teilweise geschlossen wird.

Durch die Erfindung wird eine Vorrichtung zum Applizieren flüssiger Arzneimittel geschaffen, bei der Druckgas verwendet wird, um das als Vorrat in einem flexiblen Beutel gehaltene flüssige Arzneimittel dem Patienten zuzuführen. Der Druckgasbehälter kann im druckdichten Gehäuse zusammen mit dem flexiblen Beutel untergebracht werden, wobei ein sehr kleiner Druckgasbehälter ausreicht, beispelsweise wie er von Taschenfeuerzeugen, Siphongeräten und dergleichen bekannt ist. Wird die abgegebene Menge des flüssigen Arzneimittels von einem an den Beutel angeschlossenen Ventil gesteuert, genügt es, zu Beginn des Betriebes der Vorrichtung das Auslassventil des Druckgasbehälters zu öffnen, wodurch sich im druckdichten Gehäuse ein betimmter Druck einstellt, der sich auch nach längerer Betriebszeit nicht abbaut, wenn das die Vorrichtung umschliessende Gehäuse tatsächlich druckdicht ist. Andererseits kann man aber auch durch Steuern des Gasdruckes im druckdichten Gehäuse die abgegebenen und zu applizierenden Mengen des flüssigen Arzneimittels steuern, wenn dies aus irgendwelchen Gründen zweckmässiger sein sollte als die Steuerung durch ein in der Auslassleitung des flexiblen Beutels vorgesehenes Steuerventil vorzunehmen.

Zweckmässig ist die vom flexiblen Beutel nach aussen führende Leitung eine Kapillare mit genau definiertem Querschnitt, so dass sich die Menge des abgegebenen flüssigen Arzneimittels genau steuern lässt, und zwar gleichgültig ob in der Auslassleitung des flexiblen Beutels ein Steuerventil vorgesehen ist oder die Steuerung über Druckveränderungen im druckdichten Gehäuse erfolgt. Im Falle eines Steuerventiles in der Auslassleitung kann die Steuerung der pulsierend oder kontinuierlich abgegebenen Menge des flüssigen Arzneimittels auch durch den Öffnungsgrad des Steuerventils vorgenommen werden.

Die Steuerventile sind zweckmässig elektrisch oder elektronisch mittels einer Batterie zu betätigen, wobei als Batterie eine Minizelle ausreicht, wie man sie beispielsweise bei elektronisch betriebenen Uhren, Hörhilfen und dergleichen kennt. Derartige Batterien nehmen wenig Platz in Anspruch und sind sehr leicht, so dass sie die Grösse und das Gewicht der Vorrichtung nicht wesentlich beeinflussen.

Zweckmässig werden die Steuerventile mittels Mikroprozessoren betätigt, d.h. mittels Steuerelementen, die sehr klein und leicht sind und wenig elektrische Energie verbrauchen. Um die erfindungsgemässe Vorrichtung an eine Applikationsleitung bzw. eine Applikationsnadel anschliessen zu können, ist nach einem weiteren Merkmal der Erfindung auf der Aussenseite des druckdichten

3  **0 098 893**  4

Gehäuses ein Anschlussstück zum lösbaren Anschliessen eines dem Patienten implantierten Schlauches vorgesehen. Der Schlauch bzw. die an diesem angebrachte Applikationsnadel braucht nicht ausgewechselt zu werden, wenn die erfindungsgemässe Vorrichtung ausgewechselt wird, was von Zeit zu Zeit erforderlich ist, wenn der Vorrat des flüssigen Arzneimittels und/oder das Druckgas erschöpft ist. Unter Umständen kann jedoch auch eine Nachfüllbarkeit des flexiblen Beutels und/oder des Druckgasbehälters vorgesehen sein.

Die Steuerventile sind ebenso wie der Druckgasbehälter und der flexible Beutel vorzugsweise im druckdichten und druckbeständigen Gehäuse angeordnet, so dass dieses Gehäuse die Vorrichtung nach aussen abschliesst. Besonders in diesem Falle kann die Vorrichtung als implantierbare Baueinheit ausgebildet sein.

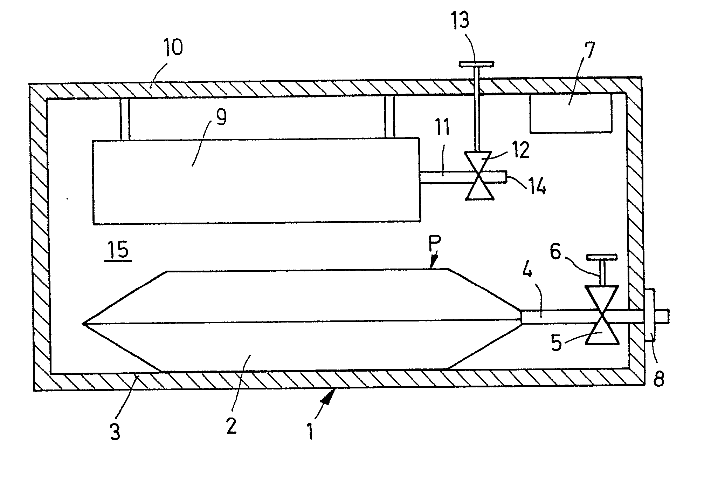

In der Zeichnung ist ein Ausführungsbeispiel der erfindungsgemässen Vorrichtung schematisch dargestellt.

Die Vorrichtung hat ein druckdicht ausgebildetes kastenartiges Gehäuse 1, das die in ihm untergebrachten Teile nach aussen hermetisch abkapselt. In dem Gehäuse 1 ist ein flexibler Beutel 2 angeordnet, der zur Aufnahme eines Vorrates eines flüssigen Arzneimittels dient und beim dargestellten Ausführungsbeispiel auf dem Boden 3 des Gehäuses 1 liegt. Der Beutel 2 kann dort in nicht dargestellter Weise befestigt sein, jedoch ist es auch möglich, ihn lose in das Gehäuse 1 einzulegen.

Vom Beutel 2 geht eine Leitung 4 aus, die zur Abgabe des im Beutel 2 befindlichen Arzneimittels dient. Diese Leitung 4 ist eine Kapillare mit definiertem Lumen bzw. definiertem offenen Querschnitt, um ein genau dosierbares Abgeben von flüssigem Arzneimittel zu ermöglichen. Die Leitung 4 kann ein flexibler Schlauch oder auch eine starre Leitung sein. Im letztgenannten Falle müsste sie gegenüber dem Gehäuse 1 abgestützt werden, um ein Abknicken zu verhindern.

In der Leitung 4 ist innerhalb des Gehäuses 1 ein Steuerventil 5 vorgesehen, das über eine nicht näher dargestellte Steuereinrichtung 6, beispielsweise Mikroprozessoren, steuerbar ist, so dass das Steuerventil 5 bei pulsatorischer Zugabe des Arzneimittels während der Applikationszeit geöffnet und dann wieder geschlossen wird. Als Energiequelle ist im Gehäuse 1 eine elektrische Batterie 7 in Form einer Minizelle angebracht.

Auf der Aussenseite des Gehäuses 1 ist ein Adapter 8 befestigt, der zum Anschliessen einer nicht dargestellten Schlauchleitung oder dergleichen dient, die das flüssige Arzneimittel eine Infusionsnadel, die einem Patienten implantiert ist, zuführt.

Im Gehäuse 1 ist ausserdem ein Druckgasbehälter 9 angebracht, der beim dargestellten Ausführungsbeispiel an der oberen Wand 10 befestigt ist. Dieser Druckgasbehälter 9 ist mit einer Auslassleitung 11 versehen, in der ein Steuerventil 12 liegt, das über einen Handgriff 13 zu betätigen, insbesondere zu öffnen ist. Bei geöffnetem Steuerventil 12 strömt das im Druckgasbehälter 9 befindliche Gas durch eine Auslassöffnung 14 in den Innenraum 15 des Gehäuses 1 und übt dann einen Druck P auf den flexiblen Beutel 2 aus. Ist das Steuerventil 5 ganz oder teilweise geöffnet, wird entsprechend dem Druck P flüssiges Arzneimittel aus dem flexiblen Beutel 2 durch die Leitung 4 herausgedrückt.

Die in der Zeichnung dargestellte Vorrichtung benötigt als Energiequelle lediglich eine sehr kleine und leichte Batterie 7 und einen Druckgasbehälter 9. Der Druckgasbehälter 9 kann von Hand geöffnet werden, so dass hierfür noch nicht einmal eine Energiequelle notwendig ist. Vielmehr genügt es, wenn der behandelnde Arzt, welcher die Vorrichtung an den Patienten anschliesst, das Steuerventil 12 öffnet, um den Betriebsbeginn der Vorrichtung einzuleiten. Es ist aber auch eine automatische Steuerung des Steuerventils 12 möglich, ebenso wie dies in Verbindung mit dem Steuerventil 5 beschrieben ist.

**Patentansprüche**

1. Vorrichtung zum Applizieren flüssiger Arzneimittel, mit einem das zu applizierende Arzneimittel aufnehmenden flexiblen Beutel (2) und einer von diesem ausgehenden Leitung (4) zum Abgeben des Arzneimittels, wobei der Beutel (2) in einem druckdichten, mit einem unter Druck stehenden Gas zu füllenden Gehäuse (1) angeordnet und jeweils ein Steuerventil (5; 12) zum Steuern des Gasdruckes im Gehäuse bzw. der aus dem Beutel abgegebenen Menge des Arzneimittels in der Leitung (4) vorgesehen ist, dadurch gekennzeichnet, dass im druckdichten Gehäuse (1) ein Vorratsbehälter (9) für Druckgas, der ein steuerbares Auslassventil (12) aufweist, und die Steuerventile (5; 12) angeordnet sind.

2. Vorrichtung nach Anspruch 1, dadurch gekennzeichnet, dass die vom flexiblen Beutel (2) nach aussen führende Leitung (4) ein Kapillar mit genau definiertem Querschnitt ist.

3. Vorrichtung nach einem der Ansprüche 1 bis 3, dadurch gekennzeichnet, dass die Steuerventile (5; 12) elektrisch oder elektronisch mittels einer im Gehäuse (1) angebrachten Batterie (7) zu betätigen sind.

4. Vorrichtung nach Anspruch 3, dadurch gekennzeichnet, dass die Steuerventile (5; 12) mittels Mikroprozessoren zu betätigen sind.

5. Vorrichtung nach einem der Ansprüche 1 bis 4, dadurch gekennzeichnet, dass sie als implantierbare Baueinheit ausgebildet ist.

**Claims**

1. Device for administering liquid medicaments comprising a flexible bag (2) for receiving the medicament to be administered and a duct (4) connected thereto for supplying the medicament, said bag (2) being located in pressure-tight casing (1) to be filled with pressurized gas, said duct (4) being provided with pilot valves (5; 12) for controlling the gas pressure in said housing, respectively

3

for controlling the amount of medicament supplied from said bag, characterized in that said pressure-tight casing (1) comprises a pressure-gas storage reservoir (9) provided with a controllable discharge valve (12) and that pilot valves (5; 12) are located in said casing.

2. Device as defined by claim 1, characterized in that duct (4) outwardly extending from flexible bag (2) is a capillary of precisely defined section.

3. Device as defined by either of claims 1 to 3, characterized in that pilot valves (5; 12) are controlled electrically or electronically by means of a battery (7) located in casing (1).

4. Device as defined by claim 3, characterized in that pilot valves (5; 12) are controlled by means of microprocessors.

5. Device as defined by either of claims 1 to 4, characterized in that they are designed as implantable construction unit.

**Revendications**

1. Dispositif pour administrer des médicaments liquides, comportant un sachet souple (2) qui contient le médicament à administrer et une conduite (4) qui en sort pour distribuer les médicaments, étant précisé que le sachet (2) est disposé dans un boîtier (1) étanche à la pression et que l'on doit remplir de gaz sous pression et qu'il est respectivement prévu un robinet de commande (5, 12) pour commander la pression du gaz qui règne dans le boîtier et la quantité de médicament distribuée dans la conduite (4) à partir du sachet, caractérisé en ce que, dans le boîtier étanche à la pression (1), sont disposés un réservoir de gaz sous pression (9) qui présente un robinet de sortie (12) qui peut se commander, ainsi que les robinets de commande (5, 12).

2. Dispositif selon la revendication 1, caractérisé en ce que la conduite (4) qui conduit du sachet souple (2) à l'extérieur est une conduite capillaire d'une section définie avec précision.

3. Dispositif selon l'une des revendications 1 et 2, caractérisé en ce que les robinets de commande (5, 12) sont à manœuvrer par voie électrique ou électronique au moyen d'une batterie (7) rapportée dans le boîtier (1).

4. Dispositif selon la revendication 3, caractérisé en ce que les robinets de commande (5, 12) sont à manœuvrer au moyen de microprocesseurs.

5. Dispositif selon l'une des revendications 1 à 4, caractérisé en ce qu'il est conçu sous forme d'ensemble implantable.